# EUROPEAN PATENT APPLICATION

(11) **EP 4 505 976 A1**
(43) Date of publication of application: **12.02.2025**
(21) Application number: 22939479.6
(22) Date of filing: 18.05.2022
(51) Int. Cl.: A61F 2/24

(54) **VALVE DELIVERY SYSTEM**

(30) Priority: 25.04.2022 CN 202210444096
(71) Applicant: Kingstronbio (Changshu) Co., Ltd., Changshu Jiangsu 215500 (CN)
(72) Inventor: ZHANG, Bo, Changshu, Jiangsu 215500 (CN); ZHONG, Shengping, Changshu, Jiangsu 215500 (CN); MENG, Chunwang, Changshu, Jiangsu 215500 (CN)
(74) Representative: Wu, Ting
(86) International application number: PCT/CN2022/093472
(87) International publication number: WO 2023/206645

(57) **Abstract**

A valve delivery system. The valve delivery system comprises a valve and a delivery device, wherein the valve is provided with a fixing part (1). The fixing part (1) is provided with a fixing hole (11). The delivery device comprises a bolt wire assembly (2) and a withdrawing assembly (3). The bolt wire assembly (2) comprises a bolt wire (21). The withdrawing assembly (3) comprises a fixing claw (31). The fixing claw (31) is provided with a limiting hole (32), and the fixing claw (31) has elasticity. When the fixing part (1) passes through the limiting hole (32), the bolt wire assembly (2) can pass through the fixing hole (11) and limit the separation of the fixing part (1) from the limiting hole (32), so that the valve is in a connected state. When the bolt wire (21) is separated from the fixing hole (11), the fixing part (1) is separated from the limiting hole (32) under the elastic action of the valve, so that the valve is in an unhooked state. The valve is controlled by means of the fixing claw (31) with the elasticity and the bolt wire (21), so that the delayed and controllable release of the valve can be realized, thereby reducing the displacement of the valve and the risk of injuring a human body caused by too fast deformation of the valve during release.

## Description

### TECHNICAL FIELD

The present disclosure relates to the technical field of medical devices, and in particular, to a valve delivery system.

### BACKGROUND

An artificial valve is used in fields such as heart valve diseases. It is a medical device implanted into the heart to replace a heart valve and achieve unidirectional blood flow. A self-expanding valve may be curled up and contracted at low temperatures, and when entering the body, it may be automatically expanded. The valve is delivered to an appropriate position in the body by means of a delivery device outside the body, and then is released. However, the valve itself has a certain elasticity, and the valve is prone to pop out and undergo large deformation at a moment when the valve is separated from the delivery device, which might lead to an overall displacement of the valve and even damage the physiological structure of a human body.

### SUMMARY

The present disclosure provides a valve delivery system to solve the problems of the valve displacement and damage to the human body when the valve is released.

The present disclosure provides a valve delivery system, including a valve provided with a fixing part, and a delivery device. The delivery device includes a bolt wire assembly, including a bolt wire extending along an axial direction of the delivery device; and a withdrawing assembly, including a fixing claw. The fixing claw is elastic, the fixing claw is provided with a limiting hole, and the fixing part is able to pass through the limiting hole. When the fixing part passes through the limiting hole and the bolt wire passes through a fixing hole of the fixing part, the valve is able to be partially expanded under an action of its own elastic force, the fixing part is unable to be separated from the limiting hole, and the valve is in a connected state; or when the bolt wire is separated from the fixing hole, the fixing part is able to be separated from the limiting hole, and the valve is in an unhooked state.

The valve delivery system provided by the present disclosure is used for placing a valve in a patient's body. The valve may be a self-expanding valve. After being delivered to a preset position, the self-expanding valve may be automatically expanded to play a supporting role once it is released from fixation. The valve includes a fixing part for fixing the valve into the delivery device. For example, the fixing part is provided with a fixing hole. The delivery device is used to deliver the valve to the human body. At least a part of the fixing part of the valve may pass through a limiting hole. At this time, the bolt wire may pass through the fixing hole of the valve, so that the fixing part is unable to be separated from the limiting hole, and the valve is in a connected state. When the delivery device delivers the valve to the preset position in the body and releases the valve, the valve is gradually expanded under the action of its own deformation elastic force until it drives the fixing claw to entirely open. At this time, the bolt wire assembly may be pulled so that the bolt wire is separated from the limiting hole. Under the action of its own elastic force, the fixing part of the valve may be separated from the limiting hole and thus expanded and opened, and the valve is in an unhooked state. The fixing claw and the bolt wire are elastic. During the gradual release of the valve, the valve may drive the fixing claw to expand outward under the action of its own elastic force. The bolt wire may displace along the radial direction of the withdrawing assembly. The valve may be partially expanded first. When the shape and position of the valve meet the preset requirements, the bolt wire assembly may be pulled to make the bolt wire separated from the fixing hole to completely release the valve, so as to achieve the delayed and controllable release of the valve. When the current valve and delivery device release the valve and the valve is separated from the delivery device, due to the elasticity of the valve itself, the valve will be fully expanded instantly. This relatively rapid and large deformation may cause the valve to displace or even damage the human body, thus affecting the use effect. In addition, since the valve needs to be loaded into a delivery device having a relatively small volume and then delivered to the body, a size of the valve in the connected state is significantly different from a size of the valve in the unhooked state, and a connecting structure needs to be provided to fix the valve. If a long claw structure is provided at the valve, the size of the valve will be increased, affecting the structural design of the valve and even increasing the risk of lesions. The delivery device provided by the embodiments of the present disclosure can release the valve in a delayed and controllable manner, and reduce the possibility of valve displacement or damage to the human body when the valve deforms during release.

In a possible implementation manner, the withdrawing assembly is provided with a through hole, and the bolt wire is able to pass through the through hole and cooperate with the fixing hole.

The bolt wire assembly is able to move along the axial direction of the delivery device. The bolt wire assembly is arranged at a side of the withdrawing assembly away from the valve. The through hole penetrates through the withdrawing assembly along the axial direction of the delivery device. The bolt wire may pass through the through hole and extend into the fixing hole of the valve, thereby restricting the valve from being unhooked and keeping the valve in a connected state. When the bolt wire assembly moves in a direction away from the withdrawing assembly, the bolt wire may move within the through hole and thus be separated from the fixing hole. The valve is no longer limited by the bolt wire and may be expanded under the action of its own elastic force. The through hole is correspondingly arranged with the fixing claw. The through hole may play a guiding role for the bolt wire, so that the bolt wire may accurately cooperate with the fixing hole of the valve, thereby reducing the possibility of the bolt wire being offset.

In a possible implementation manner, the withdrawing assembly is provided with an avoidance part, and the avoidance part is correspondingly arranged with the fixing claw and is in communication with the through hole.

The avoidance part is arranged at a side of the fixing claw of the withdrawing assembly close to the axial direction of the withdrawing assembly. The avoidance part is recessed towards the axis of the withdrawing assembly. When the fixing part extends into the limiting hole, the fixing part may be bended towards the axis of the withdrawing assembly. At least a part of the fixing part may extend into the avoidance part, so that the fixing hole may cooperate with the bolt wire.

In a possible implementation manner, an end of the fixing claw is connected to an end of the withdrawing assembly close to the bolt wire assembly, and another end of the fixing claw is provided with the limiting hole, a size of the another end of the fixing claw provided with the limiting hole is larger than a size of the end of the fixing claw connected to the end of the withdrawing assembly close to the bolt wire assembly.

An end of the fixing claw is fixed at an end of the withdrawing assembly close to the bolt wire assembly, and another end of the fixing claw extends to an end of the withdrawing assembly away from the bolt wire assembly. This arrangement may extend an overall length of the fixing claw and improve the elasticity of the fixing claw. At the end of the fixing claw provided with the limiting hole, a larger size may allow the limiting hole to have a sufficient setting area without increasing a volume of other parts of the fixing claw. When releasing the valve, the fixing claw has better elasticity to fix the valve, so that the valve can reach a partially expanded state first, thereby realizing the release control of the valve.

In a possible implementation manner, the withdrawing assembly includes a plurality of fixing claws, the bolt wire assembly is correspondingly provided with a plurality of bolt wires, and the plurality of fixing claws are arranged along a circumferential direction of the withdrawing assembly.

The plurality of fixing claws are arranged along the circumferential direction of the withdrawing assembly, which may fix the valve simultaneously in multiple directions, thereby ensuring stable fixation of the valve and an evenly distributed force on the valve. When releasing the valve, the bolt wire assembly can drive the plurality of bolt wires to be separated from the fixing holes at the same time. The plurality of fixing claws simultaneously release the restriction on the valve, so that the valve may be evenly expanded, thereby reducing the possibility of the valve being offset during expansion caused by an unbalanced force.

In a possible implementation manner, the withdrawing assembly is provided with an installation groove, the fixing claw is arranged in the installation groove, the installation groove is in communication with the avoidance part, and the installation groove is correspondingly arranged with the through hole.

The installation groove is correspondingly arranged with the fixing claw, and the installation groove is in communication with the avoidance part. When the valve is in a connected state, at least a part of the fixing claw may be embedded in the installation groove, so that the fixing claw does not protrude relative to the surface of the withdrawing assembly, which is convenient for the storage of the withdrawing assembly, thereby facilitating the delivery device to deliver the valve into the human body. The limiting hole may be located in the avoidance part, which is convenient for the fixing part to extend into the limiting hole and cooperate with the bolt wire.

In a possible implementation manner, the delivery device includes a wire removal device and a wire removal connecting tube, an end of the wire removal connecting tube is connected to the bolt wire assembly, and another end of the wire removal connecting tube is connected to the wire removal device. The wire removal device is able to move along the axial direction of the delivery device.

The wire removal device is provided with a handle and is connected to the bolt wire assembly through a wire removal connecting tube. When the delivery device delivers the valve into the human body, it is convenient for controlling the movement of the bolt wire device by the wire removal device, thereby realizing the movement control of the bolt wire to release the valve.

In a possible implementation manner, the delivery device includes an outer tube and a wheel assembly, the wheel assembly is connected to the outer tube, and the wheel assembly is able to move along the axial direction of the delivery device and is configured to drive the outer tube to move to release the valve.

The outer tube is used for fixing and storing the valve. The wheel assembly is arranged at an end of the delivery system far away from the valve. The wheel assembly includes threads. When the wheel assembly rotates, the wheel assembly may move along the axial direction of the delivery device, thereby driving the outer tube to move and release the restriction on the valve, and thus releasing the valve. Using the wheel assembly for control can improve the release precision of the outer tube.

In a possible implementation manner, the delivery device includes a locking device, the locking device is able to lock the wire removal device and restrict the wire removal device from moving along the axial direction of the delivery device.

The locking device is arranged at an end of the wire removal device far away from the bolt wire assembly. The locking device may include a locking groove and a locking member. A part of the locking member may extend into the locking groove, and another part of the locking member protrudes relative to the locking groove, thereby restricting the wire removal device from moving in the direction away from the bolt wire assembly. After the locking member is separated from the locking groove, the locking of the wire removal device is released. The locking device can reduce the possibility of misoperation and reduce the risk of the valve being released prematurely.

In a possible implementation manner, the delivery device includes an introduction device, the introduction device is provided with a protruding part, the protruding part is arranged around a side wall of the introduction device, and a shape of the protruding part is the same as a shape of an end contour of the valve after contraction.

The introduction device includes a conical tip, which is convenient for entering into the human body easily. The introduction device includes a protruding part arranged around the tip, and the shape of the protruding part is arc-shaped, which is consistent with the shape of the valve in the connected state, thereby being convenient for the delivery device to store the valve and can release the valve more compliantly. An opening of the outer tube may also be provided as a corresponding arc-shaped opening to cooperate with the protruding part of the introduction device, which facilitates the introduction device to introduce the delivery device into the human body.

The present disclosure provides a valve delivery system. The valve delivery system includes a valve and a delivery device. The valve is provided with a fixing part. The fixing part is provided with a fixing hole. The delivery device includes a bolt wire assembly and a withdrawing assembly. The bolt wire assembly includes a bolt wire. The withdrawing assembly includes a fixing claw. The fixing claw is provided with a limiting hole. When the fixing part passes through the limiting hole, the bolt wire assembly can pass through the fixing hole to restrict the fixing part from being separated from the limiting hole, so that the valve is in a connected state. When the bolt wire is separated from the fixing hole, the fixing part is separated from the limiting hole under an action of an elastic force of the valve, and the valve is in an unhooked state. The valve is controlled by means of the fixing claw with the elasticity and bolt wire, so that the delayed and controllable release of the valve can be realized, thereby reducing the displacement of the valve and the risk of injuring a human body caused by too fast deformation of the valve during release.

It should be understood that the above general description and the following detailed description are only exemplary and cannot limit the present disclosure.

### BRIEF DESCRIPTION OF DRAWINGS

Fig. 1 is a schematic structural diagram of a valve delivery system provided by the present disclosure;
Fig. 2 is a partial enlarged view of the part I in Fig. 1;
Fig. 3 is a schematic structural diagram of a withdrawing assembly provided by the present disclosure;
Fig. 4 is a schematic structural diagram of a bolt wire assembly provided by the present disclosure;
Fig.5 is an assembly diagram of a valve delivery system provided by the present disclosure; and
Fig. 6 is a schematic structural diagram of an introduction device provided by the present disclosure.

Reference signs:
1 - fixing part;
11 - fixing hole;
2 - bolt wire assembly;
21 - bolt wire;
3 - withdrawing assembly;
31 - fixing claw;
32 - limiting hole;
33 - through hole;
34 - avoidance part;
35 - installation groove;
4 - wire removal device;
5 - wire removal connecting tube;
6 - outer tube;
7 - wheel assembly;
8 - locking device;
9 - introduction device;
91 - protruding part.

The accompanying drawings herein are incorporated into the description and constitute a part of the description, showing embodiments conforming to the present disclosure and being used together with the description to explain the principle of the present disclosure.

### DESCRIPTION OF EMBODIMENTS

To better illustrate the technical solutions of the present disclosure, the following will describe the embodiments of the present disclosure in conjunction with the accompanying drawings in detail.

It should be clear that the described embodiments are only some embodiments of the present disclosure, not all embodiments of the present disclosure. Based on the embodiments in the present disclosure, all other embodiments obtained by those of ordinary skill in the art without creative efforts shall fall within the protection scope of the present disclosure.

The terms used in the embodiments of the present disclosure are merely for the purpose of describing particular embodiments but not intended to limit the present disclosure. Unless otherwise noted in the context, the singular form expressions "a", "an", "the" and "said" used in the embodiments and appended claims of the present disclosure are also intended to represent plural form expressions thereof.

It should be understood that the term "and/or" used herein is merely an association relationship describing associated objects, indicating that there may be three relationships, for example, A and/or B may indicate that three cases, i.e., A existing individually, A and B existing simultaneously, B existing individually. In addition, the character "/" herein generally indicates that the related objects before and after the character form an "or" relationship.

It should be noted that, the expressions such as "upper", "lower", "left", "right" and the like mentioned in embodiments of the present disclosure are described with reference to the placement status in the accompanying drawings, and should not be construed as limiting embodiments of the present disclosure. In addition, it should also be understood that, in the context, while referring to an element being formed "above" or "below" another element, it is possible that the element is directly formed "above" or "below" the other element, it is also possible that the element is formed "above" or "below" the other element via an intermediate element.

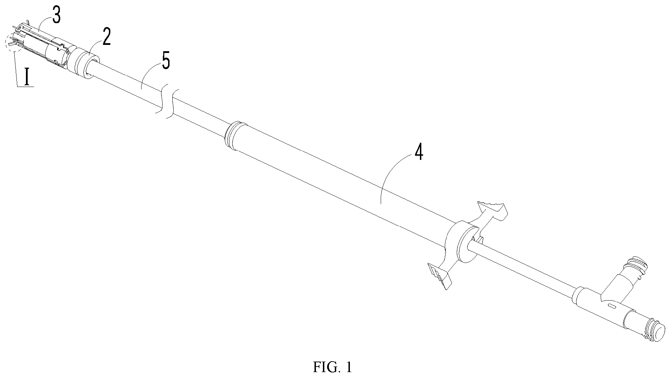

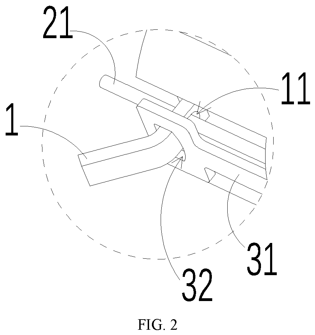

As shown in Figs. 1 and 2, an embodiment of the present disclosure provides a valve delivery system. The valve delivery system includes a valve provided with a fixing part 1, and a delivery device. The delivery device includes a bolt wire assembly 2 and a withdrawing assembly 3. The bolt wire assembly 2 includes a bolt wire 21. The bolt wire 21 extends along an axial direction of the delivery device. The withdrawing assembly 3 includes a fixing claw 31. The fixing claw 31 is provided with a limiting hole 32. The fixing part 1 is able to pass through the limiting hole 32. The fixing claw 31 is elastic. When the fixing part 1 passes through the limiting hole 32 and the bolt wire 21 passes through a fixing hole 11 of the fixing part 1, the valve is able to be partially expanded under an action of its own elastic force, the fixing part 1 is unable to be separated from the limiting hole 32, and the valve is in a connected state. When the bolt wire 21 is separated from the fixing hole 11, the fixing part 1 is able to be separated from the limiting hole 32, and the valve is in an unhooked state.

The valve delivery system provided by the embodiments of the present disclosure is used for placing a valve in a patient's body. The valve may be a self-expanding valve. After being delivered to a preset position, the self-expanding valve may be automatically expanded to play a supporting role once it is released from fixation. The valve is provided with a fixing part 1, for fixing the valve in the delivery device. For example, the fixing part 1 is provided with a fixing hole 11. The delivery device is used to deliver the valve to the human body. At least a part of the fixing part 1 of the valve can pass through the limiting hole 32. At this time, the bolt wire 21 can pass through the fixing hole 11 of the valve, so that the fixing part 1 cannot be separated from the limiting hole 32, and the valve is in a connected state. When the delivery device delivers the valve to a preset position in the body and the valve is released, the valve is gradually expended under the action of its own deformation elastic force until it drives the fixing claw 31 to entirely open. At this time, the bolt wire assembly 2 can be pulled so that the bolt wire 21 is separated from the limiting hole 32. Under the action of its own elastic force, the fixing part 1 of the valve can be separated from the limiting hole 32 and thus expanded and opened, and the valve is in an unhooked state. The fixing claw 31 is elastic. During release, the valve can drive the fixing claw 31 to expand outwards under the action of its own elastic force. The bolt wire 21 can displace along the radial direction of the withdrawing assembly 3. The valve may be partially expanded first. Then, when the shape and position of the valve meet the preset requirements, the bolt wire assembly 2 may be pulled to make the bolt wire 21 be separated from the fixing hole 11 to completely release the valve, thereby realizing the delayed and controllable release of the valve. Currently, for a valve and a delivery device, when the valve is released and separated from the delivery device, due to the elasticity of the valve itself, the valve will be fully expanded instantly. This relatively rapid and large deformation may cause the valve to displace or even damage the human body, thus affecting the use effect. In addition, since the valve needs to be loaded into a delivery device having a relatively small volume and then delivered to the body, a size of the valve in a connected state is significantly different from a size of the valve in a unhooked state. A connecting structure needs to be provided to fix the valve. If a long claw structure is provided at the valve, a size of the valve will be increased, affecting the structural design of the valve and even increasing the risk of lesions. The delivery device provided by the embodiments of the present disclosure can release the valve in a controllable and delayed manner, and reduce the possibility of valve displacement or damage to the human body when the valve deforms during release.

As shown in Figs. 1 to 3, in a possible implementation manner, the withdrawing assembly 3 is provided with a through hole 33. The bolt wire 21 is able to pass through the through hole 33 and cooperate with the fixing hole 11.

The bolt wire assembly 2 may move along the axial direction of the delivery device. The bolt wire assembly 2 is arranged at a side of the withdrawing assembly 3 away from the valve. The through hole 33 penetrates though the withdrawing assembly 3 along the axial direction of the delivery device. The bolt wire 21 may pass through the through hole 33 and extend into the fixing hole 11 of the valve, thereby restricting the valve from being unhooked and keeping the valve in a connected state. When the bolt wire assembly 2 moves in a direction away from the withdrawing assembly 3, the bolt wire 21 may move within the through hole 33 and thus be separated from the fixing hole 11. The valve is no longer limited by the bolt wire 21 and may be expanded under the action of its own elastic force. The through hole 33 is correspondingly arranged with the fixing claw 31. The through hole 33 may play a guiding role for the bolt wire 21, so that the bolt wire 21 may accurately cooperate with the fixing hole 11 of the valve, thus reducing the possibility of the bolt wire 21 being offset.

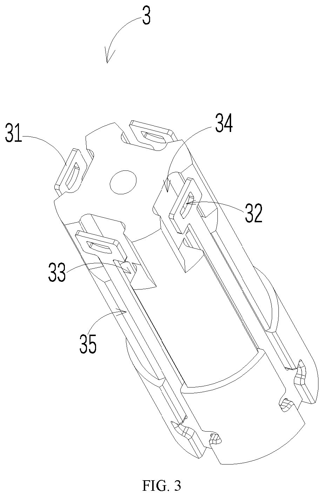

As shown in Fig. 3, in a possible implementation manner, the withdrawing assembly 3 is provided with an avoidance part 34. The avoidance part 34 is correspondingly arranged with the fixing claw 31 and is in communication with the through hole 33.

The avoidance part 34 is arranged at a side of the fixing claw 31 of the withdrawing assembly 3 close to the axial direction of the withdrawing assembly 3. The avoidance part 34 is recessed towards the axis of the withdrawing assembly 3. When the fixing part 1 extends into the limiting hole 32, the fixing part 1 may be bended towards the axis of the withdrawing assembly 3. At least a part of the fixing part 1 may extend into the avoidance part 34, so that the fixing hole 11 may cooperate with the bolt wire 21.

As shown in Fig. 3, in a possible implementation manner, an end of the fixing claw 31 is connected to an end of the withdrawing assembly 3 close to the bolt wire assembly 2, and another end of the fixing claw 31 is provided with a limiting hole 32, and a size of the another end of the fixing claw 31 provided with the limiting hole 32 is larger than a size of the end of the fixing claw 31 connected to the end of the withdrawing assembly 3 close to the bolt wire assembly.

An end of the fixing claw 31 is fixed at an end of the withdrawing assembly 3 close to the bolt wire assembly 2, and another end of the fixing claw 31 extends to an end of the withdrawing assembly 3 away from the bolt wire assembly 2. This arrangement may extend an overall length of the fixing claw 31 and increase the elasticity of the fixing claw 31. At an end of the fixing claw 31 provided with the limiting hole 32, a larger size may make the limiting hole 32 have an sufficient arrangement area without increasing a volume of other parts of the fixing claw 31. When releasing the valve, the fixing claw 31 has better elasticity to fix the valve, so that the valve may be partially expanded first, thereby realizing the delayed release control of the valve.

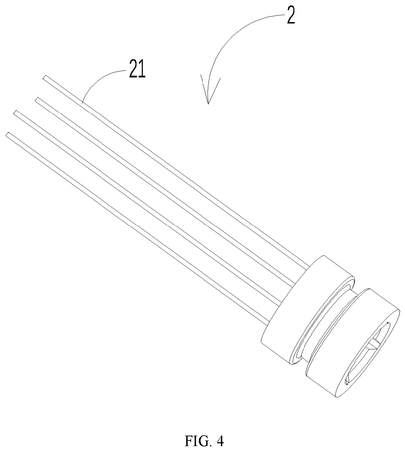

As shown in Figs. 3 and 4, in a possible implementation manner, the withdrawing assembly 3 includes a plurality of fixing claws 31, the bolt wire assembly 2 is correspondingly provided with a plurality of bolt wires 21, and the plurality of fixing claws 31 are arranged along a circumferential direction of the withdrawing assembly 3.

The plurality of fixing claws 31 are arranged along the circumferential direction of the withdrawing assembly 3, so that the valve can be fixed simultaneously in multiple directions, ensuring stable fixation of the valve and an evenly distributed force on the valve. When releasing the valve, the bolt wire assembly 2 can drive the plurality of bolt wires 21 to be separated from the fixing holes 11 at the same time. The plurality of fixing claws 31 simultaneously release the restriction on the valve, so that the valve can be evenly expanded, thereby reducing the possibility of the valve being offset during expansion caused by an unbalanced force.

As shown in Fig. 3, in a possible implementation manner, the withdrawing assembly 3 is provided with an installation groove 35, and the fixing claw 31 is arranged in the installation groove 35. The installation groove 35 is in communication with the avoidance part, and the installation groove 35 is correspondingly arranged with the through hole 33. The fixing claw 31 is fixed at an end of the withdrawing assembly 3 close to the bolt wire assembly 2.

The installation groove 35 is correspondingly arranged with the fixing claw 31, and the installation groove 35 is in communication with the avoidance part. When the valve is in a connected state, at least a part of the fixing claw 31 may be embedded in the installation groove 35, so that the fixing claw 31 does not protrude relative to the surface of the withdrawing assembly 3, which is convenient for the storage of the withdrawing assembly 3, thereby facilitating the delivery device to deliver the valve into the human body. The limiting hole 32 may be located in the avoidance part 34, which is convenient for the fixing part 1 to extend into the limiting hole 32 and cooperate with the bolt wire 21.

As shown in Fig. 1, in a possible implementation manner, the delivery device includes a wire removal device 4 and a wire removal connecting tube 5, an end of the wire removal connecting tube 5 is connected to the bolt wire assembly 2, and another end of the wire removal connecting tube 5 is connected to the wire removal device 4. The wire removal device 4 is able to move along the axial direction of the delivery device.

The wire removal device 4 is provided with a handle and is connected to the bolt wire assembly 2 through the wire removal connecting tube 5. When the delivery device delivers the valve into the human body, the wire removal device 4 is convenient for controlling the movement of the bolt wire assembly 2, thereby realizing the movement control of the bolt wire 21 to release the valve.

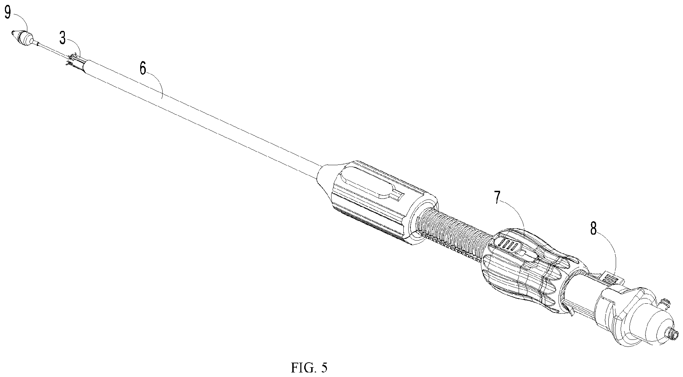

As shown in Fig. 5, in a possible implementation manner, the delivery device includes an outer tube 6 and a wheel assembly 7, the wheel assembly 7 is connected to the outer tube 6, and the wheel assembly 7 is able to move along the axial direction of the delivery device and is used to drive the outer tube 6 to move and release the valve.

The outer tube 6 is used for fixing and storing the valve. The wheel assembly 7 is arranged at an end of the delivery system far away from the valve. The wheel assembly 7 includes threads. When rotating the wheel assembly 7, the wheel assembly 7 may move along the axial direction of the delivery device, thereby driving the outer tube 6 to move and release the restriction on the valve, and thus releasing the valve. Using the wheel assembly 7 for control can improve the release precision of the outer tube 6.

As shown in Figs. 1 and 5, in a possible implementation manner, the delivery device includes a locking device 8, and the locking device 8 is able to lock the wire removal device 4 and restrict the wire removal device 4 from moving along the axial direction of the delivery device.

The locking device 8 is arranged at an end of the wire removal device 4 far away from the bolt wire assembly 2. The locking device 8 may include a locking groove and a locking member. A part of the locking member may extend into the locking groove, and another part of the locking member protrudes relative to the locking groove, thereby restricting the wire removal device 4 from moving in the direction away from the bolt wire assembly 2. After the locking member is separated from the locking groove, the locking of the wire removal device 4 is released. The locking device 8 may reduce the possibility of misoperation and reduce the risk of the valve being released prematurely.

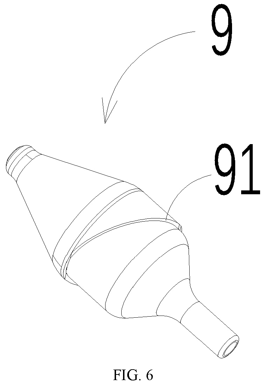

As shown in Fig. 6, in a possible implementation manner, the delivery device includes an introduction device 9, the introduction device 9 is provided with a protruding part 91, the protruding part 91 is arranged around a side wall of the introduction device 9, and a shape of the protruding part 91 is the same as an end shape of the valve after contraction.

The introduction device 9 includes a conical tip for easily entering into the human body. The introduction device 9 includes a protruding part 91 around the tip, and a shape of the protruding part 91 is arc-shaped, which is consistent with an end contour shape of the valve in the connected state, which is convenient for the delivery device to store the valve and can release the valve more compliantly. An opening of the outer tube 6 may also be provided as a corresponding arc-shaped opening to cooperate with the protruding part 91 of the introduction device 9, thereby facilitating the introduction device 9 to introduce the delivery device into the human body.

The embodiments of the present disclosure provide a valve delivery system. The valve delivery system includes a valve and a delivery device. The valve is provided with a fixing part 1. The fixing part 1 is provided with a fixing hole 11. The delivery device includes a bolt wire assembly 2 and a withdrawing assembly 3. The bolt wire assembly 2 includes a bolt wire 21. The withdrawing assembly 3 includes a fixing claw 31. The fixing claw 31 is provided with a limiting hole 32. Moreover, the fixing claw 31 is elastic. When the fixing part 1 passes through the limiting hole 32, the bolt wire assembly 2 may pass through the fixing hole 11 to restrict the fixing part 1 from being separated from the limiting hole 32, so that the valve is in a connected state. When the bolt wire 21 is separated from the fixing hole 11, the fixing part 1 is separated from the limiting hole 32 under an action of an elastic force of the valve, and the valve is in an unhooked state. The valve is controlled by means of the fixing claw 31 with the elasticity and bolt wire 21, so that the delayed and controllable release of the valve can be realized, thereby reducing the displacement of the valve and the risk of injuring a human body caused by too fast deformation of the valve during release.

The above-described embodiments are merely preferred embodiments of the present disclosure and are not intended to limit the present disclosure. Various changes and modifications can be made to the present disclosure by those skilled in the art. Any modifications, equivalent substitutions and improvements made within the principle of the present disclosure shall fall into the protection scope of the present disclosure.

## Claims

1. A valve delivery system, comprising:
a valve provided with a fixing part (1); and
a delivery device,
wherein the delivery device comprises:
a bolt wire assembly (2), comprising a bolt wire (21) extending along an axial direction of the delivery device; and
a withdrawing assembly (3), comprising a fixing claw (31), wherein the fixing claw (31) is elastic, the fixing claw (31) is provided with a limiting hole (32), and the fixing part (1) is able to pass through the limiting hole (32);
wherein when the fixing part (1) passes through the limiting hole (32) and the bolt wire (21) passes through a fixing hole (11) of the fixing part (1), the valve is able to be partially expanded under an action of its own elastic force, the fixing part (1) is unable to be separated from the limiting hole (32), and the valve is in a connected state; or when the bolt wire (21) is separated from the fixing hole (11), the fixing part (1) is able to be separated from the limiting hole (32), and the valve is in an unhooked state.

2. The valve delivery system according to claim 1, wherein the withdrawing assembly (3) is provided with a through hole (33), and the bolt wire (21) is able to pass through the through hole (33) and cooperate with the fixing hole (11).

3. The valve delivery system according to claim 2, wherein the withdrawing assembly (3) is provided with an avoidance part (34), and the avoidance part (34) is correspondingly arranged with the fixing claw (31) and is in communication with the through hole (33).

4. The valve delivery system according to claim 1, wherein an end of the fixing claw (31) is connected to an end of the withdrawing assembly (3) close to the bolt wire assembly (2), and another end of the fixing claw (31) is provided with the limiting hole (32); and a size of the another end of the fixing claw (31) provided with the limiting hole (32) is larger than a size of the end of the fixing claw (31) connected to the end of the withdrawing assembly (3) close to the bolt wire assembly (2).

5. The valve delivery system according to claim 1, wherein the withdrawing assembly (3) comprises a plurality of fixing claws (31), the bolt wire assembly (2) is correspondingly provided with a plurality of bolt wires (21), and the plurality of fixing claws (31) are arranged along a circumferential direction of the withdrawing assembly (3).

6. The valve delivery system according to claim 3, wherein the withdrawing assembly (3) is provided with an installation groove (35), the fixing claw (31) is arranged in the installation groove (35), the installation groove (35) is in communication with the avoidance part (34), and the installation groove (35) is correspondingly arranged with the through hole (33).

7. The valve delivery system according to any one of claims 1 to 6, further comprising a wire removal device (4) and a wire removal connecting tube (5),
wherein an end of the wire removal connecting tube (5) is connected to the bolt wire assembly (2), and another end of the wire removal connecting tube (5) is connected to the wire removal device (4); and
wherein the wire removal device (4) is able to move along the axial direction of the delivery device.

8. The valve delivery system according to any one of claims 1 to 6, further comprising an outer tube (6) and a wheel assembly (7), wherein the wheel assembly (7) is connected to the outer tube (6), and the wheel assembly (7) is able to move along the axial direction of the delivery device and is configured to drive the outer tube (6) to move to release the valve.

9. The valve delivery system according to claim 7, further comprising a locking device (8), wherein the locking device (8) is able to lock the wire removal device (4) and restrict the wire removal device (4) from moving along the axial direction of the delivery device.

10. The valve delivery system according to any one of claims 1 to 6, further comprising an introduction device (9), wherein the introduction device (9) is provided with a protruding part (91), the protruding part (91) is arranged around a side wall of the introduction device (9), and a shape of the protruding part (91) is the same as a shape of an end contour of the valve after contraction.
